Europäisches Patentamt

**European Patent Office**

**Office européen des brevets**

(19)

(11) Publication number: **0 128 467**
**A1**

# EUROPEAN PATENT APPLICATION

(12)

(21) Application number: **84106214.4**

(22) Date of filing: **30.05.84**

(51) Int. Cl.³: **C 07 C 103/52,** C 12 N 15/00,
C 12 P 21/00, A 61 K 45/00
// C12R1/19

(30) Priority: **01.06.83 US 499964**

(71) Applicant: **F. HOFFMANN-LA ROCHE & CO.**
**Aktiengesellschaft, CH-4002 Basel (CH)**

(43) Date of publication of application: **19.12.84**
**Bulletin 84/51**

(72) Inventor: **DeChiara, Thomas M., 42 Summit Avenue,**
**Bloomfield, N.J. (US)**
Inventor: **Tarnowski, Stanley Joseph, Jr., 115 Vreeland**
**Avenue, Nutley, N.J. (US)**

(84) Designated Contracting States: **AT BE CH DE FR GB IT**
**LI LU NL SE**

(74) Representative: **Lederer, Franz, Dr. et al, Patentanwälte**
**Dr. Lederer Franz Meyer-Roxlau Reiner F.**
**Lucile-Grahn-Strasse 22, D-8000 München 80 (DE)**

(54) Polypeptides having interferon activity.

(57) Polypeptides having interferon activity with an amino acid sequence corresponding to the amino acid sequence of an interferon selected from a rIFN-α, a hybrid rIFN-α, rIFN-β and rIFN-γ in which at least one cysteine residue has been replaced by an amino acid residue which is incapable of forming an intermolecular disulfide bond; DNA sequences coding for these polypeptides; replicable plasmidic expression vehicles containing these DNA sequences; microorganisms transformed with these expression vehicles; and methods of preparing them.

F. Hoffmann-La Roche & Co. Aktiengesellschaft 0128467
Basel / Schweiz

30 Mai 1984

RAN 4100/36

— 1 —

## Polypeptides having Interferon Activity

Interferons are proteins which are synthesized by cells in response to viral infection, immune stimulation and a variety of inducers and which are presently divided into three major classes: alpha or leukocyte interferon (IFN-α), beta or fibroblast interferon (IFN-ß) and gamma or immune interferon (IFN-γ). The interferons have anti-viral and antiproliferative activities, a potent ability to confer a virus-resistant state in targeted cells and immu-nomodulatory activities (J.L. Toy, Clin. exp. Immunol. 54, 1-13 [1983]). Their biological properties have led to the clinical use of interferons as therapeutic agents for the treatment of viral infections and malignancies.

Interferons have been obtained from natural sources such as leukocytes derived from the buffy coats of whole blood, lympoblastoid cells in continuous suspension culture or fibroblast cultures upon stimulation with inducing agents. Cells which can be stimulated to produce IFN-γ include both B and T cells. The interferons obtained from these sources have been purified to homogeneity. Interferons have also been produced using methods of recombinant DNA technology, i.e. by expression from microorganisms transformed with expression vectors containing interferon genes under the control of suitable promoter-operator sequences. The leukocyte, fibroblast and immune interferons produced by recombinant techniques are designated rIFN-α, rIFN-ß and rIFN-γ, respectively. Since at least 12 distinct IFN-α genes have been identified the corresponding proteins are designated rIFN-αA, rIFN-αB, rIFN-αC and so forth. The amino acid sequences of rIFN-αA-L, as well as the nucleotide sequences which

Mez/8.5.84

encode them are described, e.g., by Pestka in Archiv. Biochem. Biophys. 221, 1 (1983) and in British Patent Specification No. 2 079 291.

Goeddel and coworkers achieved the initial expression of rIFN-αA in E. coli cells transformed with the recombinant plasmid pL 31 (Nature, 287, 411-416 [1980]). This plasmid contains the structural gene for mature rIFN-αA (i.e., a gene in which the nucleotide sequence encoding a 23-amino acid signal peptide normally translated in the human cell has been removed and an ATG "start" signal has been inserted immediately before the codon for the first amino acid following the signal peptide) under the control of an appropriately positioned promoter-operator sequence. The rIFN-α interferons are 165 amino acids (in the case of rIFN-αA) or 166 amino acids in length, except that they may, in some instances contain an additional methionine attached to the N-terminus of the ordinarily first amino acid of the protein as the result of translation of the ATG start signal which encodes methionine.

Hybrid leukocyte interferons have been produced by expression of genes which are produced by cleaving two or more IFN-α genes at internal endonuclease cleavage sites and then ligating one or more fragments of one gene with one or more fragments of a different gene (or genes) to produce a new gene encoding a complete 165- or 166-amino acid IFN-α with specific segments corresponding to portions of different IFN-α species. In this manner, for example, it has been possible to produce a leukocyte interferon in which the amino acid sequence corresponds to amino acids 1-91 of rIFN-αA followed by amino acids 92-166 of rIFN-αD. Similarly, it has been possible to produce a hybrid IFN-α in which the amino acid sequence corresponds to amino acids 1-92 of rIFN-αD followed by amino acids 93-165 of rIFN-αA (J. Biol. Chem. 257, 11497-11502 [1982]).

The cloning and expression of mature rIFN-ß, its amino acid structure, as well as the nucleotide sequence coding therefor are described by Goeddel et al. in Nucleic Acids Research 8, 4057 (1980).

The cloning and expression of mature rIFN-γ, its amino acid structure and the nucleotide sequence coding therefor are described by Gray et al. in Nature 295, 503 (1982).

However, allelic variations of the specific amino acid sequences of the above-cited interferons are possible and have already been described. For example, Nagata and coworkers described the expression of an rIFN-α gene (denoted as rIFN-$\alpha_1$) which yielded a polypeptide (in non-mature form) differing from rIFN-αD by a single amino acid at position 114 (Nature 284, 316 [1980]). Similarly the cloning and expression of another rIFN-α gene (identified as rIFN-$\alpha_2$) which yielded a polypeptide differing from rIFN-αA by a single amino acid at position 23 has been described in European Patent Application No. 32 134; published July 15, 1981.

A problem which has occurred in the manufacture and use of interferons is that the individual interferon molecules tend to oligomerize. While the etiology of these oligomers has not been completely understood it is believed that the methods used to purify interferons for therapeutic use such as high pressure liquid chromatography or monoclonal antibody affinity chromatography may contribute to the formation of dimers, trimers and higher oligomers of interferon. These oligomeric forms which have been observed particularly with respect to leukocyte and fibroblast interferons result from two or more interferon molecules becoming irreversibly associated with one another through intermolecular covalent bondings, such as disulfide linkages.

While the dimeric forms of interferons are believed to retain biological activity, the higher oligomeric forms in many cases have either no biological activity or significantly reduced activity by comparison to the monomeric forms. Moreover, the oligomeric forms have the potential for causing deleterious side effects if used therapeutically.

All of the known rIFN-αs, rIFN-ß and rIFN-γ contain several cysteine residues the sulfhydryl side-chains of which are capable of forming intramolecular and intermolcular disulfide bonds, which latter result in oligomerization. The amino acid sequence of rIFN-αA contains cysteine residues at positions 1, 29, 98 and 138. Wetzel assigned intramolecular disulfide bonds between the cysteine residues at positions 1 and 98 and between the cysteine residues at positions 29 and 138 (Nature 289, 606 [1981]).

Heretofore, efforts to overcome the oligomerization problem have concentrated on adjusting the purification conditions in order to prevent the formation of oligomers or on post-processing reaction conditions whereby intermolecular disulfide bonds are reduced.

The present invention in a first broad aspect relates to novel polypeptides having interferon activity comprising an amino acid sequence corresponding to the amino acid sequence of an interferon selected from a rIFN-α, a hybrid rIFN-α, rIFN-ß and rIFN-γ wherein, however, at least one cysteine residue has been replaced by another amino acid residue, i.e. an amino acid residue which is incapable of forming an intermolecular disulfide bond.

In a preferred embodiment of this broad aspect, the invention relates to a polypeptide with the amino acid sequence of rIFN-αA in which, however, at least one of the cysteine residues at positions 1 and 98 has been replaced by another amino acid residue which is incapable of forming an intermolecular disulfide bond.

The invention also relates to pharmaceutical compositions with antiviral activity containing the novel polypeptides and which are essentially free of oligomers other than dimers and, preferably, contain only a stable monomeric interferon.

In a second broad aspect, this invention relates to methods and intermediates for producing the novel polypeptides described above by recombinant DNA technology. In particular, this aspect relates to a method of producing a double stranded DNA (dsDNA) encoding the novel polypeptides by using restriction enzymes to excise a portion of the dsDNA comprising the codon for the undesired cysteine residue from the parental interferon gene and replacing it with a synthetic oligodeoxynucleotide segment wherein the nucleotide triplet coding for cysteine has been replaced by a nucleotide triplet coding for another amino acid residue which is incapable of forming an intermolecular disulfide bond.

Accordingly, this second broad aspect of the invention encompasses dsDNA sequences coding for the novel polypeptides, replicable plasmidic expression vehicles containing these dsDNA sequences, microorganisms transformed with these replicable plasmidic expression vehicles and a method of preparing these dsDNA sequences.

Preferred polypeptides of the invention are those obtained by substitution of cysteine residues in rIFN-αs and hybrid rIFN-αs. All rIFN-αs and corresponding hybrid rIFN-αs contain a cysteine residue at the N-terminus, i.e. at position 1. Additionally, rIFN-αA contains cysteine residues at positions 29, 98 and 138, rIFN-αB at

positions 29, 100 and 139; rIFN-αC, F, G, H, I, J, K and L at positions 29, 99 and 139; and rIFN-αD at positions 29, 86, 99 and 139. It has to be noted that Pestka, in Archiv. Biochem. Biophys. 221, 1 (1983), Fig. 17, shows cysteine residues at positions 99 and 139 of rIFN-αA rather than at positions 98 and 138. This is because the sequence representation has been shifted one position following position 43 to align it with the other rIFN-α species which contain one more amino acid residue than rIFN-αA. All amino acid positions recited herein refer to sequentially numbered amino acids and do not account for such shifts.

It is understood that the polypeptides of the invention have amino acid sequences corresponding to amino acid sequences of known rIFNs wherein, however, at least one cysteine residue has been replaced by another amino acid residue which is incapable of forming intermolecular disulfide bonds. The cysteine residues which are replaced are those which are not required to maintain interferon activity of the molecule. As used herein the term "interferon activity" refers to antiviral and antigrowth activity characteristic of the interferons. The characteristic antiviral activity of rIFN can be determined using the cytopathic effect inhibition test described by Familletti et al. in Methods in Enzymology 78, 387 (1981). The characteristic antigrowth activity of rIFN can be determined using the procedure described by Evinger, M. & Pestka, S., Methods in Enzymology 79, 45 (1981).

Since the intramolecular disulfide bridge between the cysteine residues at positions 29 and 138 of rIFN-αA is believed to be required for interferon activity, these residues have not to be replaced. Similarly, the cysteine residues at positions 29 and 139 of rIFN-αB through L (and allelic variations thereof) should not be replaced.

It has been shown by Shepard et al. (Nature 294, 563--565 [1981]) that substitution of the cysteine at position 141 of rIFN-ß results in a loss of biological activity. Accordingly, this cysteine residue is not to be replaced.

In accordance with the present invention the cysteine residue at position 1 of rIFN-αA has been replaced as well as both cysteine residues at positions 1 and 98 of rIFN-αA, without any concomitant loss of interferon activity. Analogously, the cysteine residue(s) at position 1 and/or position 99 (in the case of rIFN-αC through L) and position 1 and/or 100 (in the case of rIFN-αB) can be replaced. In the case of a hybrid rIFN-α, cysteine residues corresponding to these positions, depending upon the parental rIFN and the segment used, may be replaced. In the case of rIFN-αD or a hybrid rIFN-α containing an amino acid sequence of rIFN-αD comprising the cystein residue at position 87 this cystein residue may also be replaced.

The amino acid residues which replace the cysteine residues of the parental interferons in the polypeptides of the invention can be any amino acid residue which is incapable of participating in the formation of an intermolecular disulfide bond. In the case of the N-terminal cysteine residue, i.e. the residue at position 1, this is preferably replaced with glycine. In the case of internal cysteine residues, e.g. the residue at position 98 of rIFN-αA, these are preferably replaced with serine, since the serine side chain most closely resembles the spatial arrangement of the cysteine side chain. Thus, substitution with serine presents the least possibility for disrupting the conformation of the molecule.

While rIFN-αs, hybrid rIFN-αs, and rIFN-γ possess a cysteine residue at position 1, the interferon activity is retained even if this cysteine residue is deleted. Novel polypeptides of this type can be prepared using recombinant DNA methodology by which the portion of the parental interferon gene which encodes the N-terminus is replaced by a synthetic oligodeoxynucleotide sequence wherein the codon for the first cystein residue is missing in a manner as described in Example 1 hereinafter. In this case the ATG translation initiation signal would immeditely be followed by the nucleotide triplet encoding the amino acid residue at position 2 of the parental interferon. The resultant dsDNA can be incorporated into an expression vehicle which can be used to transform a microorganism. The transformant microorganism can then be grown up and under suitable reaction conditions will express the novel polypeptide having interferon activity the amino acid sequence of which is that of a des(Cys 1)-rIFN-α, a hybrid des(Cys 1)-rIFN-α or des(Cys 1)-rIFN-γ.

In a preferred embodiment of the invention, a gene encoding rIFN-αA has been modified in a manner described in detail hereinafter and has been expressed to yield a rIFN-αA in which one or both cysteine residues at positions 1 and 98 have been replaced by amino acid residues which are incapable of forming intermolecular disulfide bonds. Accordingly, a preferred embodiment of the present invention is a polypeptide of the formula

$R^1$ ASP LEU PRO GLN THR HIS SER LEU GLY SER ARG ARG
THR LEU MET LEU LEU ALA GLN MET ARG LYS ILE SER
LEU PHE SER CYS LEU LYS ASP ARG HIS ASP PHE GLY
PHE PRO GLN GLU GLU PHE GLY ASN GLN PHE GLN LYS
ALA GLU THR ILE PRO VAL LEU HIS GLU MET ILE GLN
GLN ILE PHE ASN LEU PHE SER THR LYS ASP SER SER
ALA ALA TRP ASP GLU THR LEU LEU ASP LYS PHE TYR
THR GLU LEU TYR GLN GLN LEU ASN ASP LEU GLU ALA —

— R$^2$ VAL ILE GLN GLY VAL GLY VAL THR GLU THR PRO
LEU MET LYS GLU ASP SER ILE LEU ALA VAL ARG LYS
TYR PHE GLN ARG ILE THR LEU TYR LEU LYS GLU LYS
LYS TYR SER PRO CYS ALA TRP GLU VAL VAL ARG ALA
GLU ILE MET ARG SER PHE SER LEU SER THR ASN LEU
GLN GLU SER LEU ARG SER LYS GLU

wherein R$^1$ and R$^2$ are amino acid residues with the proviso at least one of R$^1$ and R$^2$ is an amino acid residue which is incapable of participating in the formation of an intermolecular disulfide bond.

Initially, the gene for rIFN-αA has been modified by excision of a portion of the gene encoding the N-terminus of the polypeptide including the nucleotide triplet encoding cysteine at position 1 and replacement with a synthetic oligodeoxynucleotide coding for glycine at position 1. The section of the gene containing the nucleotide triplet for the cysteine at position 98 remained unchanged. The modified gene in a plasmidic expression vehicle, was expressed in E. coli to produce a polypeptide the amino acid sequence of which corresponded to the amino acid sequence of rIFN-αA except that it contained a glycine at position 1 instead of cystein. The modified polypeptide is referred to hereinafter as rIFN-αA (Gly 1). After extraction from the cells and purification on a monoclonal antibody immunoaffinity chromatography column, the interferon thus obtained was subjected to electrophoresis on sodium dodecylsulfate polyacrylamide gel (SDS-PAGE). The interferon separated into a major band, corresponding to monomeric material, and a minor band, corresponding to dimeric material. A rIFN-αA expressed from the unmodified rIFN-αA gene was electrophoresed on the same gel and separated into several bands, corresponding to the monomer, the dimer and oligomers of higher aggregation, i.e. a trimer, tetramer, etc.

The product containing a major proportion (more than half) of monomeric rIFN-αA(Gly 1) and a minor proportion of dimeric rIFN-αA(Gly 1) displayed the full antiviral activity characteristics of rIFN-αA on MDBK cells.

The gene containing the synthetic oligodeoxynucleotide segment encoding glycine at position 1 was then further modified by excision of a segment of the gene including the codon for cysteine at position 98 and replacement with an analogous synthetic oligodeoxynucleotide sequence coding for serine at position 98. This modified gene was expressed in E. coli to produce a polypeptide the amino acid sequence of which corresponded to the amino acid sequence of rIFN-αA except that it contained glycine at position 1 and serine at position 98. The modified polypeptide is referred to hereinafter as rIFN-αA(Gly 1, Ser 98). After extraction from cells and purification by a monoclonal antibody immunoaffinity chromatography column, the interferon thus obtained was subjected to SDS-PAGE. The interferon migrated as a single band, indicating the presence of essentially only monomeric material.

The product consisting essentially of monomeric rIFN-αA(Gly 1, Ser 98) displayed antiviral activity which was about the same as that of rIFN-αA on MDBK cells.

In a preferred embodiment of this invention, the microorganisms useful as recipients in the transformation procedures and unless otherwise noted, are E. coli K-12 strain 294 as described in British Patent Publication No. 2055382A (ATCC Accession No. 31446, deposited October 28, 1978) as well as E. coli MA210 or RR1 (ATCC Accession No. 31343). However other microbial strains many of which are publicly available or are deposited and available from recognized microorganism depository institutions, such as the American Type Culture Collection (ATCC) can also be used in accordance with the teaching of the present application.

All recombinant DNA work herein was performed in compliance with applicable guidelines of the National Institutes of Health.

The novel polypeptides of the invention can be used for the same purposes as the known interferons in the form of pharmaceutical compositions, e.g. as antiviral and antitumor agents and in the treatment of immunosupressive conditions. Dosages and dose rates may parallel those currently being used in clinical applications of the known interferons, typically about 1-200 x $10^6$ units daily. The polypeptides can be conveniently administered in parenteral dosage form. Suitable dosage forms can be prepared using known formulation methods to prepare pharmaceutically useful compositions wherein the polypeptide is admixed with a pharmaceutically acceptable carrier vehicle. A suitable dosage form will comprise an effective amount of the polypeptide together with a pharmaceutically acceptable carrier vehicle depending upon the particular patient, the mode of administration and the condition to be treated.

## BRIEF DESCRIPTION OF THE DRAWINGS

Fig. 1 is a representation of the amino acid sequence of rIFN-αA and a nucleotide sequence coding for rIFN-αA.

Fig. 2 is a schematic represention of the process used to produce a dsDNA coding for rIFN-αA(Gly 1).

Fig. 3 is a schematic representation of the process used to produce a dsDNA coding for rIFN-αA(Gly 1, Ser 98).

Fig. 4 is a schematic representation of the process used to produce a replicable plasmidic expression vehicle for the expression of rIFN-αA(Gly 1).

Fig. 5 is a schematic representation of the process used to produce a replicable plasmidic expression vehicle for the expression of rIFN-αA(Gly 1, Ser 98).

Asterisks in the figures indicate nucleotide substitutions replacing the nucleotide triplets encoding cysteine.

In the preferred embodiment of the present invention, the gene sequence encoding rIFN-αA is modified. The double stranded DNA sequence which is the basis for the expression of the modified rIFN-αA consists of a coding strand and a complementary strand, wherein the coding strand, reading from the 5'-end comprises the sequence

```
X GAT CTG CCT CAA ACC CAC AGC CTG GGT AGC AGG AGG
ACC TTG ATG CTC CTG GCA CAG ATG AGG AAA ATC TCT CTT
TTC TCC TGC TTG AAG GAC AGA CAT GAC TTT GGA TTT CCC
CAG GAG GAG TTT GGC AAC CAG TTC CAA AAG GCT GAA ACC
ATC CCT GTC CTC CAT GAG ATG ATC CAG CAG ATC TTC AAT
CTC TTC AGC ACA AAG GAC TCA TCT GCT GCT TGG GAT GAG
ACC CTC CTA GAC AAA TTC TAC ACT GAA CTC TAC CAG CAG
CTG AAT GAC CTG GAA GCC Y GTG ATA CAG GGG GTG GGG
GTG ACA GAG ACT CCC CTG ATG AAG GAG GAC TCC ATT CTG
GCT GTG AGG AAA TAC TTC CAA AGA ATC ACT CTC TAT CTG
AAA GAG AAG AAA TAC AGC CCT TGT GCC TGC GAG GTT GTC
AGA GCA GAA ATC ATG AGA TCT TTT TCT TTG TCA ACA AAC
TTG CAA GAA AGT TTA AGA AGT AAG GAA
```

wherein X and Y are nucleotide triplets coding for amino acid residues with the proviso that at least one of the amino acid residues for which X and Y are coding is incapable of participating in the formation of an intermolecular disulfide bond.

In particular, the present invention provides a method of producing a double stranded DNA sequence encoding a polypeptide having interferon activity which comprises:

(a)   cleaving a dsDNA containing a sequence encoding an
      interferon selected from a rIFN-α, a hybrid rIFN-α,
      rIFN-ß and rIFN-γ with an endonuclease to produce a
      first dsDNA fragment containing a nucleotide triplet
      encoding a cysteine residue and one or more other
      cleavage fragments encoding the remainder of the interferon;

(b)   separating the first dsDNA fragment from the other
      cleavage fragments encoding the remainder of the interferon;

(c)   preparing a dsDNA sequence corresponding to the separated first fragment in which, however, the nucleotide
      triplet coding for the cysteine residue has been replaced by a nucleotide triplet coding for an amino acid
      residue which is incapable of participating in the
      formation of an intermolecular disulfide bond, said
      dsDNA sequence having ends complementary to the ends of
      the dsDNA sequence(s) encoding the remainder of the
      interferon; and

(d)   ligating said dsDNA sequences in the proper orientation
      to yield a dsDNA sequence coding for a modified IFN.

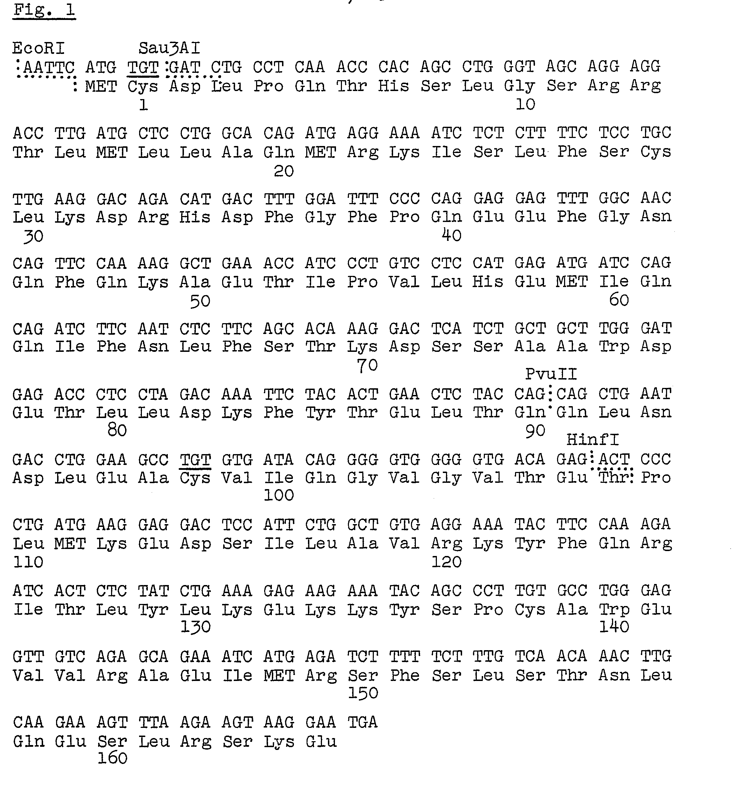

      The dsDNA encoding an rIFN which is cleaved in step (a)
above can be conveniently obtained from an expression or
cloning vector containing the IFN structural gene. As a
source of dsDNA to serve as a starting material from which
to prepare the dsDNA encoding rIFN-αA(Gly 1) and
rIFN-αA(Gly 1, Ser 98), plasmid pL 31 was employed. This
plasmid, derived from pBR322, contains a 1160 base pair
(b.p.) insert encoding a trp promoter-operator, a ribosome
binding site, an ATG translation initiation signal, the
structural gene encoding the amino acid sequence of
rIFN-αA (depicted in Fig. 1) and an untranslated
3'-sequence following the stop codon (Goeddel et al.,

Nature 287, 411 [1980]).

The endonucleases which can be used to cleave the dsDNA in step (a) are well known to those familiar with recombinant DNA technology. Which particular endonuclease is used will depend on the particular rIFN gene involved and the particular cysteine residue(s) to be replaced. It is well within the knowledge of the skilled worker to identify appropriate cleavage sites and to select a suitable endonuclease. The reaction conditions under which the cleavage reactions take place, as well as the methods for separating and purifying the fragments, are well known in the art.

In the preparation of a dsDNA sequence encoding rIFN-αA(Gly 1), advantage was taken of the existence of a Sau 3AI recognition site so that it is possible to cleave the structural gene for rIFN-αA immediately following the nucleotide triplet coding for cysteine at position 1 and to separate this codon from the remainder of the structural gene.

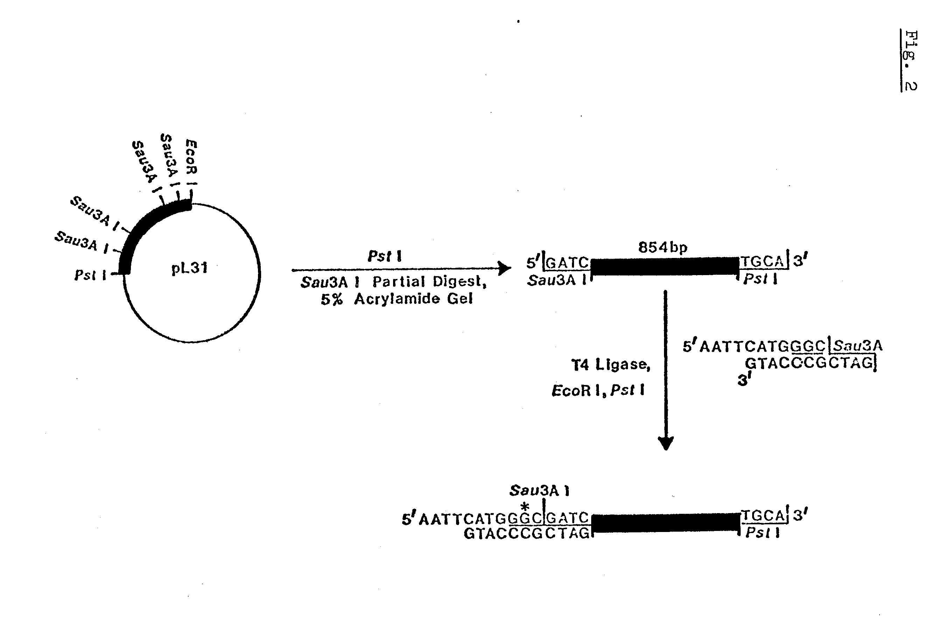

The procedure which was employed to produce a dsDNA encoding rIFN-αA(Gly 1) can be described as follows, with specific reference to Fig. 2. The plasmid pL 31, which contained the structural gene for rIFN-αA, was digested completely with Pst I and partially with Sau 3AI. An 854 b.p. fragment was isolated. The coding strand of this fragment, beginning at the Sau 3AI site contained the full DNA sequence encoding rIFN-αA except the TGT codon for cysteine at position 1 and terminated at the Pst I site 360 bases beyond the TGA stop codon.

The 5'-end of the rIFN-αA gene thus removed was replaced by ligating the 854 b.p. segment with the synthetic oligodeoxynucleotide

```
                    EcoRI          Sau 3AI
         5'     | AATTCATGGGC |
         3'           | GTACCCGCTAG |
```

wherein the GGC-codon coding for glycine replaces the TGT-codon coding for the cysteine residue in the unmodified rIFN-αA gene. The 865 b.p. dsDNA thus produced contained the sequence encoding rIFN-αA(Gly 1) and terminated with an Eco RI site at the 5'-end of the coding strand and with a Pst I site at the other end.

In order to modify the rIFN gene to replace an internal cysteine residue, e.g. the cysteine residue at position 98 of rIFN-αA or IFN-αA(Gly 1), the dsDNA encoding the rIFN can be cleaved at a first endonuclease cleavage site upstream (i.e. toward the 5'-end of the coding strand) of the nucleotide triplet encoding the undesired cysteine and at a second endonuclease cleavage site downstream (i.e. toward the 3'-end of the coding strand) of the nucleotide triplet encoding the undesired cysteine to isolate a fragment containing the undesired nucleotide triplet. The fragment containing the undesired nucleotide triplet is separated from the dsDNA encoding the remainder of the rIFN. A synthetic dsDNA sequence is prepared which corresponds to the removed segment except that the nucleotide triplet encoding cysteine is replaced by a nucleotide triplet encoding a different amino acid residue, preferably serine. This synthetic dsDNA sequence is then ligated in the proper orientation to the dsDNA encoding the remainder of the rIFN.

If it is desired to delete two internal cysteine residues which are sufficiently close together, this may be done by cleaving the gene at a first endonuclease cleavage site upstream of the nucleotide triplet encoding the first undesired cysteine residue and at a second endonuclease cleavage site downstream of the nucleotide triplet encoding the second undesired cysteine and replacing the segment thus removed with a corresponding synthetic dsDNA sequence encoding an amino acid sequence in which both cysteines are replaced by different amino acids.

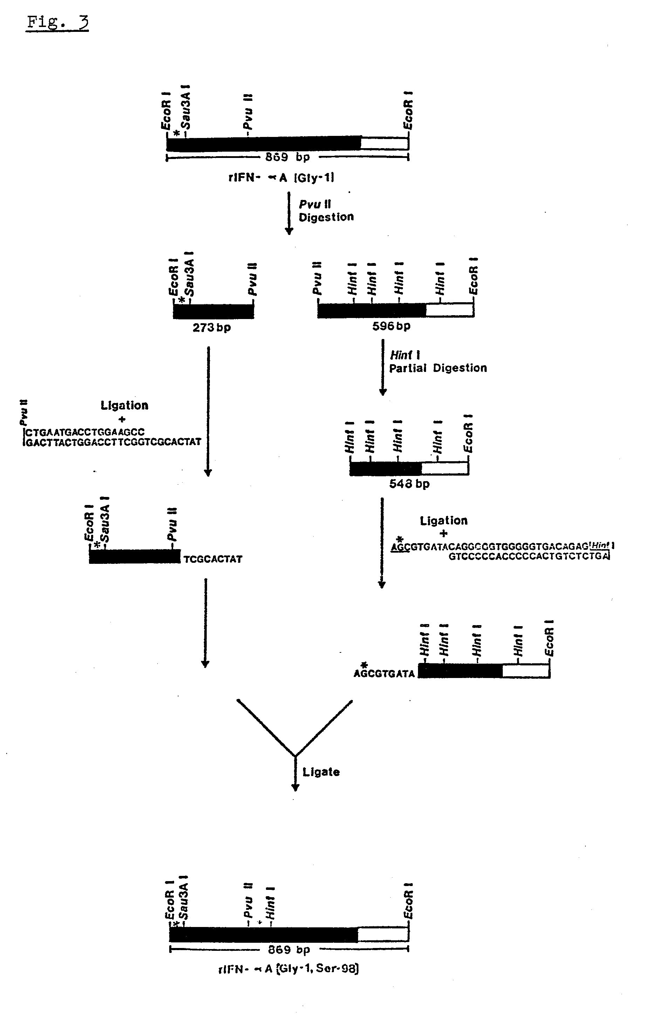

With reference to Fig. 3 the following procedure to produce a dsDNA encoding rIFN-αA(Gly 1, Ser 98) was used. The starting material employed was an 869 b.p. dsDNA encoding rIFN-αA(Gly 1). This was the 865 b.p. dsDNA produced by the previously described procedure to which additional 4 base pairs had been ligated to the 3'-end in order to create a terminal Eco RI recognition site useful for insertion into an expression vector. This dsDNA was cleaved with Pvu II, resulting in a 273 b.p. segment containing the 5'-end of the coding strand for rIFN-αA (Gly 1) and a 596 b.p. segment containing the 3'-end of the coding strand for rIFN-αA(Gly 1) including the nucleotide triplet encoding cysteine at position 98. The 596 b.p. segment was partially digested with Hinf I to produce a 48 b.p. fragment containing the nucleotide triplet encoding the cysteine at position 98 and a 548 b.p. fragment encoding the remainder of the 3'-end. Two synthetic double stranded DNA sequences (blocks I and II) were prepared to replace the 48 b.p. region of the following sequences:

```
5'    |CTGAATGACCTGGAAGCC
3'    |GACTTACTGGACCTTCGGTCGCACTAT
```

Pvu II        Block I

```
                                          Hinf I
5'    AGCGTGATACAGGGGGTGGGGGTGACAGAG|
3'              GTCCCCCACCCCCACTGTCTCTGA|
```

Block II

The AGC nucleotide triplet encoding serine in block II replaces the TGT triplet encoding cysteine in the deleted 48 b.p. fragment. Block I was ligated to the 273 b.p. fragment and block II was ligated to the 548 b.p. fragment of the original structural gene. The resulting two fragments were then ligated via their 9-base long sticky ends to produce an 869 b.p. dsDNA containing a coding strand which encoded rIFN-αA(Gly 1, Ser 98).

The dsDNAs encoding the novel polypeptides of the invention can be incorporated into an expression vehicle which can be used to transform a host microorganism for the purpose of expressing the novel polypeptides. Any of the known and commonly employed expression vectors may be used for this purpose, particularly plasmidic expression vectors.

Incorporation of the dsDNA into an expression vector can be achieved in accordance with methods well known in the art. The vector is first linearized by cutting it with an endonuclease at a cleavage site which is appropriately positioned with respect to a promoter-operator sequence directing the expression of the inserted structural gene. The dsDNA encoding the novel polypeptide is then ligated at either end to the cleaved ends of the vector to recircularize the vector. Of course, the ends of the dsDNA insert must be complementary to the cleaved ends of the vector to allow ligation. If necessary, the dsDNA ends can be rendered complementary by building up or cleaving back the ends, using known procedures, provided that the structural gene itself and the associated initiation and termination codons are left intact and that the gene remains in the proper positions and reading frame with respect to the promoter--operator sequence.

Accordingly, in accordance with the teachings of the present invention, there is provided a replicable plasmidic expression vehicle comprising:

(a) a replicon;

(b) a promoter-operator sequence;

(c) a DNA sequence encoding a ribosome binding site including a translation initiation signal and

(d) a DNA sequence, in phase with said promoter-operator sequence, coding for a polypeptide having interferon activity, said polypeptide comprising the amino acid sequence of an interferon selected from a rIFN-α, a hybrid rIFN-α, rIFN-ß and rIFN-γ wherein, however, at least one cysteine residue has been replaced by an amino acid residue which is incapable of forming intermolecular disulfide bonds, including a translation termination signal.

Any of the known plasmidic expression vectors can be employed in the preparation of the replicable plasmidic expression vehicle of this invention. Preferably plasmid pRC 23 can be employed, an expression vector which is derived from pBR 322 and contains the $P_L$ promoter-operator sequence (Bernard, H.U. and Helinski, D.R., Methods in Enzymology 68, 482). Details concerning the construction of the cloning and expression vector pRC 23 can be obtained from European Patent Application No. 83106730.1. In this vector, the strong $P_L$ promoter is controlled by the λ cI repressor. The gene coding for the repressor carries a mutation which renders the repressor temperature-sensitive. At 30°C the repressor functions normally and from about 37°C to about 42°C it is inactivated. Thus the $P_L$-promoter is repressed (turned off) at 30°C and depressed (turned on) at 42°C. The ability to control the $P_L$-promoter allows one to grow the culture at about 30-36°C without expression of the gene product and then to raise the temperature to about 37-42°C to produce the desired gene product. Plasmid pRC 23 is designed to accept dsDNA sequences which terminate in Eco RI recognition sites. Accordingly, the 869 b.p. dsDNA sequences previously mentioned, which encode either rIFN-αA(Gly 1) or rIFN-αA(Gly 1, Ser 98) and terminate in Eco RI recognition sites at either end, can be inserted directly into pRC 23 which has been linearized by cleavage with Eco RI. Ligation is carried out under known conditions using a known ligase, e.g. T4 DNA ligase.

The replicable plasmidic expression vehicle is used to transform a microorganism, preferably E. coli, to produce a transformant which is capable of expressing the polypeptide of the invention. Transformation can be conveniently carried out using known procedures such as treating the host cells with $CaCl_2$ at about 4°C. The transformed microorganism can be grown up under known fermentation conditions and the novel polypeptide expressed in the microorganism. The polypeptide is then recovered, for example, by lysing the cells and purified by known techniques. Purification can preferably be effected by immunoaffinity chromatography on a column with monoclonal antibodies to the corresponding parental rIFN which are bound to a solid support. A suitable monoclonal antibody which can be used · in the purification of rIFN-αA(Gly 1) or rIFN-αA(Gly 1, Ser 98) is described, e.g., by Staehelin et al. in Journal of Biological Chem. 256, 9750 (1981).

When the expression vector employed to produce the replicable plasmidic expression vehicle is pRC 23, the resultant transformant microorganism can be grown up to a desired density at a temperature of 30°C and the temperature can then be raised to about 42°C to inactivate the repressor protein and initiate expression.

The following examples illustrate but do not limit the present invention. Unless otherwise stated, all parts and percents are by weight and all temperatures are centigrade.

## Example 1

### Preparation of rIFN-αA(Gly 1)

(a) Preparation of dsDNA Encoding rIFN-αA (Gly 1)

Plasmid pL 31 (6 µg) containing the gene encoding rIFN-αA (Nature 287, 411-416 [1980]), was digested completely with Pst I and partially with Sau 3AI. Both digests were performed at 37°C using 20 units Pst I for 1 hr. followed by 5 units of Sau 3AI for 5 min. in a 20 µl reaction. The cleavage fragments were separated in a 1.5% agarose gel. A 854 b.p. fragment was isolated which began with the nucleotide triplet encoding Asp at position 2 of the rIFN-αA and terminated at the Pst I site 360 b.p. beyond the translation termination codon.

Using the procedure of Miyoshi et al. (Nucleic Acids Res. 8, 5507-5517 [1980]) the synthetic oligodeoxynucleotides

5' AATTCATGG<u>GGC</u>

3' GTACCCGCTAG

were prepared and the 5' ends phosphorylated. The phosphorylated oligodeoxynucleotides were ligated to the Sau 3AI site of the truncated rIFN-αA DNA fragment (100 ng) in a volume of 7 µl at 15°C for 16 hours in the presence of T4 ligase. After ligation, the ligase was inactivated by incubation at 70°C for 10 min. and the DNA was digested with 50 units of Eco RI and 10 units of Pst I for 2 hours at 37°C in 20 µl in order to regenerate the "sticky ends". The resultant 865 b.p. dsDNA fragment, which contained the coding sequence for rIFN-αA(Gly 1), had an Eco RI site at the 5'-end and a Pst I site at the 3'-end of the coding region. The fragment was purified through a 1.5% agarose gel and about 50 ng were recovered for cloning.

(b) <u>Preparation of a Replicable Plasmidic Expression Vehicle for Producing rIFN-αA (Gly 1)</u>

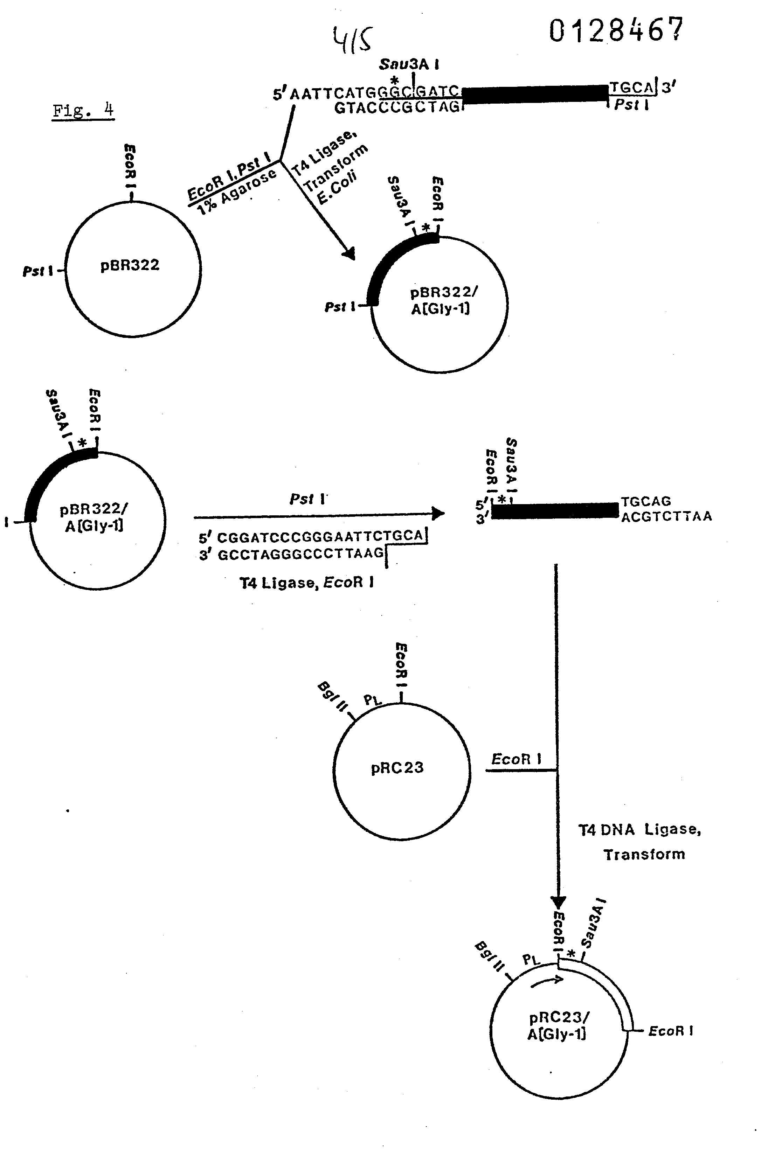

Fig. 4 is a schematic representation of the procedure which was employed to prepare the expression vehicle. We employed plasmid pRC 23 as the expression vector to prepare the replicable plasmidic expression vehicle for producing rIFN-αA(Gly 1). As previously indicated, this plasmid was derived from pBR 322 and contained a $P_L$ promoter-operator. Since pRC 23 was designed to accept genes on an Eco RI restriction site, it was necessary to convert the 3' Pst I site of the 865 b.p. fragment to an Eco RI site. In order to have a sufficient amount of the fragment to perform the modification, we first cloned the 865 b.p. fragment, using plasmid pBR 322, which contains a tetracycline resistance gene, as a cloning vector. We digested 500 ng of pBR 322 to completion at 37°C, with 10 units each of Eco RI and Pst I in a 20 µl reaction. The linearized vector thus produced was purified through a 1.0% agarose gel. The 865 b.p. fragment encoding rIFN-αA (Gly 1) (50 ng) was ligated with 100 ng of the linearized pBR 322 using T4 ligase in 10 µl for 8 hours at 15°C. After ligation, the mixture was incubated at 70°C for 10 min. and used to transform a competent MC 1061 strain of E. coli cells. The cells were plated out on LB agar containing 10 µ/ml of tetracycline and the tetracycline resistant colonies obtained from incubation at 37°C were screened for the presence of the plasmid pBR 322/rIFN-αA (Gly 1). Plasmid screening was performed by the alkaline lysis procedure of Birnboim, H.C. and Doly, J. [Nucleic Acids Res. 7, 1513 (1979)].

Synthetic oligodeoxynucleotides having the sequences

```
                              Eco RI           Pst I

      5'      CGGATCCCGGGAATTCTGCA
      3'      GCCTAGGGCCCTTAAG
```

were prepared by the procedure described by Miyoshi, et al. [Nucleic Acids Res. 8, 5507-5517 (1980)] and phosphorylated with ATP in the presence of polynucleotide kinase at the 5' ends.

The plasmid pBR 322/rIFN-αA(Gly 1) (200 ng), which contained the 865 b.p. insert encoding rIFN-αA(Gly 1), was digested to completion with 20 units of Pst I at 37°C for 1 hour and the linearized vector was purified through a 1.0% agarose gel and recovered from the gel. The synthetic oligodeoxynucleotides (50 ng each) were ligated to 200 ng of the linearized vector in a volume of 5 μl at 15°C for 16 hours. After ligase inactivation, the resultant dsDNA was digested with 50 units of Eco RI for 3 hours at 37°C. A 869 b.p. fragment encoding rIFN-αA(Gly 1) and terminating in an Eco RI site at either end, was purified on 1.0% agarose gel and recovered for cloning into the Eco RI site of pRC 23.

About 500 ng of pRC 23 was digested to completion with 10 units of Eco RI at 37°C for 1 hour in 20 μl. There was added 1 μg of calf intestinal alkaline phosphatase to dephosphorylate the Eco RI ends of the plasmid and incubation was continued for an additional 30 min. The reaction was terminated by heating at 68°C for 10 min. and the linearized vector was purified through a 1.0% agarose gel and recovered. The linearized vector (100 ng) was ligated with 50 ng of the 869 b.p. fragment encoding rIFN-αA(Gly 1) in 10 μl at 15°C for 10 hours to produce the replicable plasmidic expression vehicle pRC 23/rIFN-αA(Gly 1).

(c) Preparation of Transformant Containing pRC 23/rIFN-αA (Gly 1)

The ligation mixture was used to transform an RR1 strain of E. coli cells which contained the compatible plasmid pRK 248 cIts as described by Bernard, H.U. and Helinski, D.R. [Methods in Enzymology, 68, 482]. The compatible plasmid encodes the production of the repressor protein which recognizes and binds the operator portion of the $P_L$ promoter-operator on pRC 23. The E. coli cells were transformed at 4°C for 30 min. in the presence of 50 mmol $CaCl_2$. The cells were plated out at 30°C on LB agar containing ampicillin (50 µg/ml). The pRC 23 plasmid contains a gene for ampicillin resistance. After incubation the ampicillin resistant colonies were selected and screened for the presence of the plasmid pRC 23/rIFN-αA (Gly 1) with the gene inserted in the proper orientation relative to the $P_L$ promoter.

(d) <u>Expression and Purification of rIFN-αA (Gly 1)</u>

A colony of transformants containing the plasmid pRC 23/rIFN-αA(Gly 1) and the compatible plasmid was grown up in 2 ml of LB agar containing ampicillin (50 µg/ml) at a temperature which was not allowed to exceed 30°C. When the $OD_{600}$ of the culture reached 0.6, the temperature was raised to 42°C to inactivate the repressor protein and to initiate expression. After 2 hours at 42°C the cells were harvested and lysed in 50 µl of 7M guanidine-HCl at 0°C for 10 min. Extracts were centrifuged for 5 min. at 12,000 g. The supernatants were diluted 1:100 for antiviral assay.

The rIFN-αA(Gly 1) was extracted from E. coli cell paste using an extraction buffer containing 2M guanidine-HCl, 2% Triton x 100, 0.1M Tris-Cl, pH 7.5, for 2 hours at 4°C. The extraction mixture was diluted five-fold with cold distilled water, centrifuged at 10,000 x g for 1 hour and purified on a 1 x 2-cm immunoaffinity chromatography column. The immunoaffinity column was packed with 1.0 ml of agarose gel to which there was covalently bound approximately 13 mg of a monoclonal antibody to rIFN-αA. Preparation of the monoclonal antibody, identified as Li-8, is described by Staehelin et al. in J. Biol. Chem., <u>256</u>, 9750 (1981).

The rIFN-αA(Gly 1)-containing extraction buffer (20 ml) was loaded onto the column, which was operated at a flow rate of 1.0 ml/min. or less. The column was then sequentially washed with 5 bed-volumes each of the following solutions

1.  0.286M guanidine-HCl; 0.286% Triton x-100; 0.1M Tris-Cl, pH 7.5

2.  0.5M NaCl; 0.2% Triton x-100; 0.025M Tris-Cl, pH 7.5

3.  1.0M sodium thiocyanate; 0.1% Triton x-100; 0.025M Tris-Cl, pH 7.5

4.  0.15M NaCl; 0.1% Triton x-100.

The rIFN-αA(Gly 1) was then eluted from the column using an elution solvent of 0.2M acetic acid, 0.15M NaCl and 0.1% Triton x-100 at pH 2.5.

The eluate from the immunoaffinity chromatography column was tested for antiviral activity against vesicular stomatitis virus using the cytopathic effect inhibition test described by Familletti et al., supra. The specific activity of the eluate containing the rIFN-αA/Gly 1) was $2 \times 10^8$ units/mg of protein, which corresponds to that of rIFN-αA.

Prior to raising the temperature to 42°C to initiate expression, an aliquot of the culture containing the transformants was set aside and stored in glycerol at 30°C. This aliquot was then grown up in a 10-liter fermentor containing LB agar containing ampicillin (50 μg/ml) at a temperature not exceeding 30°C until the $OD_{600}$ reached 4-5. The temperature was then raised to 42°C to initiate expression. After the $OD_{600}$ reached about 12 (2-3 hrs.), the rIFN-αA(Gly 1) was recovered and purified in a manner similar to that described above for the 2-ml culture. The purified rIFN-αA(Gly 1) obtained from the 10-liter culture had an antiviral activity on MDBK cells of $3.3 \times 10^8$ ($\pm 0.76 \times 10^8$) units/mg of protein.

The purified rIFN-αA(Gly 1) eluate from the immunoaffinity chromatography column was electrophoresed on sodium dodecylsulfate polyacrylamide gel under non-reducing conditions. While a sample of rIFN-αA, displayed bands corresponding to a slow-moving monomer, a fast-moving monomer, a dimer, a trimer and a tetramer a sample of rIFN-αA(Gly 1) displayed only slow-moving monomer and a small amount of dimer. rIFN-αA(Gly 1) which has been neutralized to pH 7.0 shows an increase in the dimer from due to pH-dependent disulfide bond formation between cysteines at position 98. A sample of rIFN-αA(Gly 1) neutralized to pH 7.0 and reduced with ß-mercaptoethanol demonstrates that the dimer is a result of disulfide bond formation.

## Example 2

## Preparation of rIFN-αA (Gly 1, Ser 98)

### 5a) Preparation of dsDNA Encoding rIFN-αA (Gly 1, Ser 98)

We prepared the dsDNA encoding rIFN-αA(Gly 1, Ser 98) by taking advantage of a Pvu II restriction site upstream of the nucleotide triplet in the rIFN-αA(Gly 1) gene encoding the cysteine at position 98 and a Hinf I restriction site downstream of this nucleotide triplet which sites are separated by 48 bases on the coding strand.

Referring to Fig. 3, we employed as a starting material pRC 23/rIFN-αA(Gly 1) which contained the 869 b.p. insert of Example 1 encoding rIFN-αA (Gly 1). The 869 b.p. segment was cleaved out of pRC 23 by digesting to completion with 20 units of Eco RI and 20 units of Pvu II for 2 hours at 37°C in 20 µl. The 869 b.p. segment was itself cleaved at the Pvu II site just upstream of the nucleotide triplet encoding cysteine at position 98 in two fragments: a 273 b.p. fragment containing the 5'-end of the rIFN-αA(Gly 1) gene and a 596 b.p. segment containing the 3'-end of the gene including the nucleotide triplet encoding cysteine at position 98. The 596 b.p. fragment was partially digested with 5 units of Hinf I at 37°C for 5 min. in 20 μl. Hinf I was then inactivated immediately at 70°C for 10 min. A 548 b.p. Hinf I-Eco RI fragment containing the 3'-end of the rIFN-αA (Gly 1) gene was isolated and purified on 1.5% agarose gel eliminating a 48 b.p. fragment containing the triplet encoding the cysteine at position 98.

Using the procedure of Miyoshi (supra) two double stranded synthetic oligodeoxynucleotides having a complementary overlapping sequence at the 3'-end of the coding strand of the first oligodeoxynucleotide and the 5'-end of the coding strand of the second oligodeoxynucleotide were prepared. When ligated at the complementary sequence, these two oligodeoxynucleotides produced a 48 b.p. insert which was identical to the 48 b.p. fragment cleaved out of the rIFN-αA(Gly 1) gene except that the nucleotide triplet encoding cysteine at position 98 was replaced by an AGC nucleotide triplet encoding serine.

The two double stranded synthetic oligodeoxynucleotides had the following nucleotide sequences:

```
5'  |CTGAATGACCTGGAAGCC
3'  |GACTTACTGGACCTTCGGTCGCACTAT
```

Pvu II      Block I

```
5'  AGCGTGATACAGGGGGTGGGGGTGACAGAG| Hinf I
3'          GTCCCCCACCCCCACTGTCTCTGA|
```

Block II

Prior to annealing the individual strands together to produce the two double strands, the 5'-ends were phosphory-

lated with ATP using polynucleotide kinase to allow ligation. Block I (50 ng) was ligated to the Pvu II site of the 273 b.p. cleavage fragment at the 5'-end of the rIFN-αA-(Gly 1) gene (300 ng) for 16 hours at 15°C in a 7 µl reaction. Block II (50 ng) was then ligated to the Hinf I site of the 548 b.p. cleavage fragment at the 3' end of the rIFN-αA(Gly 1) gene (100 ng) for 16 hours at 15°C in a 7 µl reaction. The two dsDNA fragments produced by these ligations were then ligated to each other at the 9 b.p. complementary overlapping portions of Block I and Block II for 16 hours at 15°C. The resultant 869 b.p. dsDNA encoding rIFN-αA(Gly 1, Ser 98) was isolated and purified on a 1.5% agarose gel. The dsDNA had an Eco RI recognition site at either end.

(b) Preparation of a Replicable Plasmidic Expression Vehicle for Producing rIFN-αA (Gly 1, Ser 98)

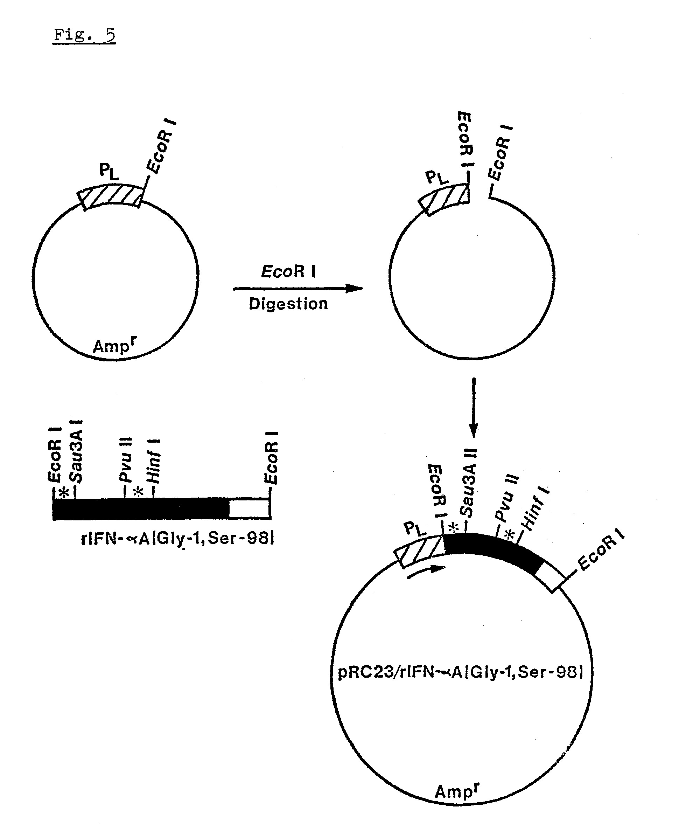

Fig. 5 is a schematic representation of the procedure which was employed to prepare the expression vehicle. Plasmid pRC 23 (500 ng) was digested to completion with 10 units of Eco RI at 37°C for 1 hour in 20 µl. There was then added 1 µg of calf intestinal alkaline phosphatase and incubation was continued an additional 30 min. The reaction was terminated by heating at 68°C for 10 min. and the linearized vector was purified through a 1.5% agarose gel and recovered. The linearized vector (100 ng) was ligated with 50 ng of the 869 b.p. fragment encoding rIFN-αA-(Gly 1, Ser 98) in 7 µl for 10 hours at 15°C to produce the replicable plasmidic expression vehicle pRC 23/rIFN-αA(Gly 1, Ser 98).

(c) Preparation of Transformant Containing pRC 23/rIFN-αA (Gly 1, Ser 98)

The ligation mixture was used to transform an RR1 strain of E. coli containing the compatible plasmid pRK 248 cIts, encoding the production of the temperature-sensitive repressor protein which recognizes and binds the operator sequence of $P_L$ promoter-operator on pRC 23. The E. coli cells were transformed at 4°C for 20 min. in the presence of 50 mmol $CaCl_2$. Ampicillin resistant colonies resulting from overnight incubation at 30°C on LB plates were selected and inoculated into 2-ml cultures of LB agar containing ampicillin (50 µg/ml). Colonies containing the recombinant plasmid pRC 23/rIFN-αA(Gly 1, Ser 98) with the gene inserted in the proper orientation relative to the $P_L$ promoter were selected by screening with the alkaline lysis method of Birnboim (supra).

(d) <u>Expression and Purification of rIFN-αA (Gly 1, Ser 98)</u>

A colony of transformants containing the plasmid pRC 23/rIFN-αA(Gly 1, Ser 98) and the compatible plasmid was grown up in 10 liters of LB agar and ampicillin (50 µg/ml) at a temperature which was not allowed to exceed 30°C. When the $OD_{600}$ of the culture reached 0.6, the temperature was raised to 42°C to inactivate the repressor protein and initiate expression. After 2 hours at 42°C the cells were harvested and lysed.

The rIFN-αA(Gly 1, Ser 98) was extracted from E. coli cell paste using 4 volumes of the buffer of Example 1(d) and purified on a 1.6 x 5.0-cm immunoaffinity chromatography column. The immunoaffinity chromatography column was packed with 10 ml of agarose gel to which there was covalently bound approximately 130 mg of a monoclonal antibody to rIFN-αA. Preparation of the monoclonal antibody, identified as Li-8, is described by Staehelin et al. in J. Biol. Chem., <u>256</u>, 9750 (1981).

The rIFN-αA(Gly 1, Ser 98)-containing extraction buffer (5,000 ml) was loaded onto the column, which was operated at a flow rate of 5.0 ml/min. or less. The column was then sequentially washed with 5 bed-volumes of the same washing solutions employed in Example 1(d). The rIFN-αA-(Gly 1, Ser 98) was then eluted from the column using the same elution solvent  as in Example 1(d).

The eluate from the immunoaffinity chromatography column was tested for antiviral activity against vesicular stomatitis virus using the cytopathic effect inhibition test described by Familletti (supra). The specific activity of the eluate containing the rIFN-αA(Gly 1, Ser 98) was $2 \times 10^8$ units/mg of protein, which corresponds to that of rIFN-αA.

The purified rIFN-αA(Gly 1, Ser 98) eluate from the immunoaffinity chromatography column was electrophoresed on sodium dodecylsulfate polyacrylamide gel under non reducing conditions. While the sample of rIFN-αA(Gly 1, Ser 98) displayed a single band of monomeric interferon a sample of rIFN-αA displayed bands corresponding to the monomer, a dimer, a trimer and higher oligomers.

CLAIMS:

1. A polypeptide having interferon activity characterized in that its amino acid sequence corresponds to the amino acid sequence of an interferon selected from a rIFN-α, a hybrid rIFN-α, rIFN-ß and rIFN-γ wherein, however, at least one cysteine residue has been replaced by an amino acid residue which is incapable of forming an intermolcecular disulfide bond.

2. A polypeptide as claimed in claim 1 which is a rIFN-α or a hybrid rIFN-α in which the cysteine residue at position 1 has been replaced by a glycine residue.

3. A polypeptide as claimed in claim 2 wherein the cysteine residue at position 98, 99 or 100 has been replaced by a serine residue.

4. A polypeptide as claimed in claim 2 or 3 wherein said rIFN-α is rIFN-αD or a hybrid rIFN-α containing a fragment of rIFN-αD including the cysteine residue at position 86 and wherein said cysteine residue at position 86 has been replaced by a serine residue.

5. A polypeptide of the formula

$R^1$ ASP LEU PRO GLN THR HIS SER LEU GLY SER ARG ARG
THR LEU MET LEU LEU ALA GLN MET ARG LYS ILE SER
LEU PHE SER CYS LEU LYS ASP ARG HIS ASP PHE GLY
PHE PRO GLN GLU GLU PHE GLY ASN GLN PHE GLN LYS
ALA GLU THR ILE PRO VAL LEU HIS GLU MET ILE GLN
GLN ILE PHE ASN LEU PHE SER THR LYS ASP SER SER
ALA ALA TRP ASP GLU THR LEU LEU ASP LYS PHE TYR
THR GLU LEU TYR GLN GLN LEU ASN ASP LEU GLU ALA —

—R² VAL ILE GLN GLY VAL GLY VAL THR GLU THR PRO
LEU MET LYS GLU ASP SER ILE LEU ALA VAL ARG LYS
TYR PHE GLN ARG ILE THR LEU TYR LEU LYS GLU LYS
LYS TYR SER PRO CYS ALA TRP GLU VAL VAL ARG ALA
GLU ILE MET ARG SER PHE SER LEU SER THR ASN LEU
GLN GLU SER LEU ARG SER LYS GLU

wherein $R^1$ and $R^2$ are amino acid residues with the proviso that at least one of $R^1$ and $R^2$ is an amino acid residue which is incapable of participating in the formation of an intermolecular disulfide bond.

6. A polypeptide as claimed in claim 5 wherein $R^1$ is Gly and $R^2$ is Cys.

7. A polypeptide as claimed in claim 5 wherein $R^1$ is Gly and $R^2$ is Ser.

8. A polypeptide as claimed in claims 1-7 wherein the first amino acid of the complete sequence is missing.

9. A polypeptide as claimed in claims 1-7 with an additional methionine residue as the amino terminus of the amino acid sequence.

10. Pharmaceutical compositions comprising a major proportion of a polypeptide with interferon activity, in monomeric form said polypeptide having an amino sequence corresponding to that of an interferon selected from a rIFN-α, a hybrid rIFN-α, rIFN-ß and rIFN-γ in which, however, at least one cysteine residue has been replaced by an amino acid residue which is incapable of forming intermolecular disulfide bonds, said composition being essentially free of oligomeric forms of the polypeptide of higher aggregation than the dimer.

11. A composition as claimed in claim 10 wherein the polypeptide is a polypeptide as claimed in any one of claims 2-9.

12. A pharmaceutical composition as claimed in claims 10 or 11 with antiviral activity.

13. A double stranded DNA sequence comprising a coding strand and a complementary strand, said coding strand encoding a polypeptide with interferon activity having an amino acid sequence corresponding to that of an interferon selected from a rIFN-α, a hybrid rIFN-α, rIFN-ß and rIFN-γ in which, however, at least one cysteine residue has been replaced by an amino acid residue which is incapable of forming intermolecular disulfide bonds.

14. A double stranded DNA sequence as claimed in claim 13 with a coding strand comprising the sequence

```
X GAT CTG CCT CAA ACC CAC AGC CTG GGT AGC AGG AGG
ACC TTG ATG CTC CTG GCA CAG ATG AGG AAA ATC TCT CTT
TTC TCC TGC TTG AAG GAC AGA CAT GAC TTT GGA TTT CCC
CAG GAG GAG TTT GGC AAC CAG TTC CAA AAG GCT GAA ACC
ATC CCT GTC CTC CAT GAG ATG ATC CAG CAG ATC TTC AAT
CTC TTC AGC ACA AAG GAC TCA TCT GCT GCT TGG GAT GAG
ACC CTC CTA GAC AAA TTC TAC ACT GAA CTC TAC CAG CAG
CTG AAT GAC CTG GAA GCC Y GTG ATA CAG GGG GTG GGG
GTG ACA GAG ACT CCC CTG ATG AAG GAG GAC TCC ATT CTG
GCT GTG AGG AAA TAC TTC CAA AGA ATC ACT CTC TAT CTG
AAA GAG AAG AAA TAC AGC CCT TGT GCC TGC GAG GTT GTC
AGA GCA GAA ATC ATG AGA TCT TTT TCT TTG TCA ACA AAC
TTG CAA GAA AGT TTA AGA AGT AAG GAA
```

wherein X and Y are nucleotide triplets coding for amino acid residues with the proviso that at least one of the amino acid residues for which X and Y are coding is incapable of participating in the formation of an intermolecular disulfide bond.

15. A double stranded DNA sequence as claimed in claim 14, wherein X is a nucleotide triplet encoding a glycine residue.

16. A double stranded DNA sequence as claimed in claim 14, wherein X is the nucleotide triplet GGC.

17. A double stranded DNA sequence as claimed in claim 14 or 15, wherein Y is a nucleotide triplet encoding a serine residue.

18. A double stranded DNA sequence as claimed in claims 14 to 17, wherein Y is the nucleotide triplet AGC.

19. A replicable plasmidic expression vehicle comprising:

(a) a replicon;
(b) a promoter-operator sequence;
(c) a DNA sequence encoding a ribosome binding site including a translation initiation signal and
(d) a DNA sequence, in phase with said promoter-operator sequence, coding for a polypeptide having interferon activity, said polypeptide comprising the amino acid sequence of an interferon selected from a rIFN-α, a hybrid rIFN-α, rIFN-ß and rIFN-γ wherein, however, at least one cysteine residue has been replaced by an amino acid residue which is incapable of forming intermolecular disulfide bonds, and a translation termination signal.

20. A replicable plasmidic expression vehicle as claimed in claim 19, wherein the polypeptide which is encoded is as claimed in any one of claims 2-8.

21. A replicable plasmidic expression vehicle as claimed in claim 19, wherein the coding strand of the DNA sequence encoding the polypeptide, reading from the 5' end, comprises a sequence as claimed in any one of claims 14-18.

22. A transformant microorganism comprising a host microorganism which has been transformed with a replicable plasmidic expression vehicle as claimed in any one of claims 19-21.

23. A transformant microorganism as claimed in claim 22 wherein the host microorganism is E. coli.

24. A process for producing a double stranded (ds) DNA sequence encoding a polypeptide having interferon activity and the amino acid sequence of an interferon selected from a rIFN-α, a hybrid rIFN-α, rIFN-ß and rIFN-γ wherein, however, at least one cysteine residue has been replaced by an amino acid residue which is incapable of forming an intermolecular disulfide bond which process comprises

(a) cleaving a dsDNA containing a sequence encoding an interferon selected from a rIFN-α, a hybrid rIFN-α, rIFN-ß and rIFN-γ with an endonuclease to produce a first dsDNA fragment containing a nucleotide triplet encoding a cysteine residue and one or more other cleavage fragments encoding the remainder of the interferon;

(b) separating the first DNA cleavage fragment containing the nucleotide triplet encoding the cysteine from the other cleavage fragments encoding the remainder of the interferon;

(c) preparing a dsDNA sequence corresponding to the separated first fragment in which, however, the nucleotide triplet coding for the cysteine residue has been replaced by a nucleotide triplet coding for an amino acid residue which is incapable of forming an intermolecular disulfide bond, said dsDNA sequence having ends complementary to the ends of the ds DNA sequence(s) encoding the remainder of the interferon; and

(d) ligating said dsDNA sequences in the proper orientation to yield a dsDNA coding for said modified interferon.

25. A process as claimed in claim 24 wherein the coding strand comprises the sequence

```
X GAT CTG CCT CAA ACC CAC AGC CTG GGT AGC AGG AGG
ACC TTG ATG CTC CTG GCA CAG ATG AGG AAA ATC TCT CTT
TTC TCC TGC TTG AAG GAC AGA CAT GAC TTT GGA TTT CCC
CAG GAG GAG TTT GGC AAC CAG TTC CAA AAG GCT GAA ACC
ATC CCT GTC CTC CAT GAG ATG ATC CAG CAG ATC TTC AAT
CTC TTC AGC ACA AAG GAC TCA TCT GCT GCT TGG GAT GAG
ACC CTC CTA GAC AAA TTC TAC ACT GAA CTC TAC CAG CAG
CTG AAT GAC CTG GAA GCC Y GTG ATA CAG GGG GTG GGG
GTG ACA GAG ACT CCC CTG ATG AAG GAG GAC TCC ATT CTG
GCT GTG AGG AAA TAC TTC CAA AGA ATC ACT CTC TAT CTG
AAA GAG AAG AAA TAC AGC CCT TGT GCC TGC GAG GTT GTC
AGA GCA GAA ATC ATG AGA TCT TTT TCT TTG TCA ACA AAC
TTG CAA GAA AGT TTA AGA AGT AAG GAA
```

wherein X and Y are nucleotide triplets coding for amino acid residues with the proviso that at least one of the amino acid residues for which X and Y are coding is incapable of participating in the formation of an intermolecular disulfide bond.

26. A process as claimed in claim 25 wherein X is a nucleotide triplet encoding a glycine residue.

27. A process as claimed in claim 25 wherein X is the nucleotide triplet GGC.

28. A process as claimed in claim 25 or 26 wherein Y is a nucleotide triplet encoding a serine residue.

29. A process as claimed in any one of claims 25 to 28 wherein Y is the nucleotide triplet AGC.

30. A process for the preparation of a polypeptide having interferon activity and the amino acid sequence of an interferon selected from a rIFN-α, a hybrid rIFN-α, rIFN-ß and rIFN-γ wherein, however, at least one cysteine residue has been replaced by an amino acid residue which is incapable of forming an intermolecular disulfide bond which process comprises culturing a host transformed by an expression vehicle containing a DNA sequence as defined in any one of claims 25-29, causing the host to express said polypeptide and recovering said polypeptide.

31. A process as claimed in claim 30 wherein said host is E. coli.

CLAIMS: (Austria)

1. A process for producing a double stranded (ds) DNA sequence encoding a polypeptide having interferon activity and the amino acid sequence of an interferon selected from a rIFN-α, a hybrid rIFN-α, rIFN-ß and rIFN-γ wherein, however, at least one cysteine residue has been replaced by an amino acid residue which is incapable of forming an intermolecular disulfide bond which process comprises

(a) cleaving a dsDNA containing a sequence encoding an interferon selected from a rIFN-α, a hybrid rIFN-α, rIFN-ß and rIFN-γ with an endonuclease to produce a first dsDNA fragment containing a nucleotide triplet encoding a cysteine residue and one or more other cleavage fragments encoding the remainder of the interferon;

(b) separating the first DNA cleavage fragment containing the nucleotide triplet encoding the cysteine from the other cleavage fragments encoding the remainder of the interferon;

(c) preparing a dsDNA sequence corresponding to the separated first fragment in which, however, the nucleotide triplet coding for the cysteine residue has been replaced by a nucleotide triplet coding for an amino acid residue which is incapable of forming an intermolecular disulfide bond, said dsDNA sequence having ends complementary to the ends of the dsDNA sequence(s) encoding the remainder of the interferon; and

(d) ligating said dsDNA sequences in the proper orientation to yield a dsDNA coding for said modified interferon.

2. A process as claimed in claim 1 wherein the coding strand comprises the sequence

```
X GAT CTG CCT CAA ACC CAC AGC CTG GGT AGC AGG AGG
ACC TTG ATG CTC CTG GCA CAG ATG AGG AAA ATC TCT CTT
TTT TCC TGC TTG AAG GAC AGA CAT GAC TTT GGA TTT CCC
CAG GAG GAG TTT GGC AAC CAG TTC CAA AAG GCT GAA ACC
ATC CCT GTC CTC CAT GAG ATG ATC CAG CAG ATC TTC AAT
CTC TTT AGC ACA AAG GAC TCA TCT GCT GCT TGG GAT GAG
ACC CTC CTA GAC AAA TTC TAC ACT GAA CTC TAC CAG CAG
CTG AAT GAC CTG GAA GCC Y GTG ATA CAG GGG GTG GGG
GTG ACA GAG ACT CCC CTG ATG AAG GAG GAC TCC ATT CTG
GCT GTG AGG AAA TAC TTC CAA AGA ATC ACT CTC TAT CTG
AAA GAG AAG AAA TAC AGC CCT TGT GCC TGC GAG GTT GTC
AGA GCA GAA ATC ATG AGA TCT TTT TCT TTG TCA ACA AAC
TTG CAA GAA AGT TTA AGA AGT AAG GAA
```

wherein X and Y are nucleotide triplets coding for amino acid residues with the proviso that at least one of the amino acid residues for which X and Y are coding is incapable of participating in the formation of an intermolecular disulfide bond.

3. A process as claimed in claim 2 wherein X is a nucleotide triplet encoding a glycine residue.

4. A process as claimed in claim 2 wherein X is the nucleotide triplet GGC.

5. A process as claimed in claim 2 or 3 wherein Y is a nucleotide triplet encoding a serine residue.

6. A process as claimed in any one of claims 2 to 5 wherein Y is the nucleotide triplet AGC.

7. A process for the preparation of a polypeptide having interferon activity and the amino acid sequence of an interferon selected from a rIFN-α, a hybrid rIFN-α, rIFN-β and rIFN-γ wherein, however, at least one cysteine residue has been replaced by an amino acid residue which is incapable of forming an intermolecular disulfide bond which process comprises culturing a host transformed by an expression vehicle containing a DNA sequence as defined in any one of claims 2-6, causing the host to express said polypeptide and recovering said polypeptide.

8. A process as claimed in claim 7 wherein said host is E. coli.

Fig. 1

```
EcoRI          Sau3AI
:AATTC ATG TGT :GAT CTG CCT CAA ACC CAC AGC CTG GGT AGC AGG AGG
:     MET Cys Asp Leu Pro Gln Thr His Ser Leu Gly Ser Arg Arg
              1                                       10

ACC TTG ATG CTC CTG GCA CAG ATG AGG AAA ATC TCT CTT TTC TCC TGC
Thr Leu MET Leu Leu Ala Gln MET Arg Lys Ile Ser Leu Phe Ser Cys
                        20

TTG AAG GAC AGA CAT GAC TTT GGA TTT CCC CAG GAG GAG TTT GGC AAC
Leu Lys Asp Arg His Asp Phe Gly Phe Pro Gln Glu Glu Phe Gly Asn
 30                                     40

CAG TTC CAA AAG GCT GAA ACC ATC CCT GTC CTC CAT GAG ATG ATC CAG
Gln Phe Gln Lys Ala Glu Thr Ile Pro Val Leu His Glu MET Ile Gln
               50                                       60

CAG ATC TTC AAT CTC TTC AGC ACA AAG GAC TCA TCT GCT GCT TGG GAT
Gln Ile Phe Asn Leu Phe Ser Thr Lys Asp Ser Ser Ala Ala Trp Asp
                            70              PvuII

GAG ACC CTC CTA GAC AAA TTC TAC ACT GAA CTC TAC CAG:CAG CTG AAT
Glu Thr Leu Leu Asp Lys Phe Tyr Thr Glu Leu Thr Gln'Gln Leu Asn
           80                                   90   HinfI

GAC CTG GAA GCC TGT GTG ATA CAG GGG GTG GGG GTG ACA GAG:ACT CCC
Asp Leu Glu Ala Cys Val Ile Gln Gly Val Gly Val Thr Glu Thr: Pro
                 100

CTG ATG AAG GAG GAC TCC ATT CTG GCT GTG AGG AAA TAC TTC CAA AGA
Leu MET Lys Glu Asp Ser Ile Leu Ala Val Arg Lys Tyr Phe Gln Arg
110                                     120

ATC ACT CTC TAT CTG AAA GAG AAG AAA TAC AGC CCT TGT GCC TGG GAG
Ile Thr Leu Tyr Leu Lys Glu Lys Lys Tyr Ser Pro Cys Ala Trp Glu
                    130                                 140

GTT GTC AGA GCA GAA ATC ATG AGA TCT TTT TCT TTG TCA ACA AAC TTG
Val Val Arg Ala Glu Ile MET Arg Ser Phe Ser Leu Ser Thr Asn Leu
                            150

CAA GAA AGT TTA AGA AGT AAG GAA TGA
Gln Glu Ser Leu Arg Ser Lys Glu
            160
```

Fig. 2

854bp

5'|GATC| ... TGCA|3'
Sau3A I ... Pst I

Pst I
Sau3A I Partial Digest,
5% Acrylamide Gel

EcoR I
Sau3A I
Sau3A I
Sau3A I
Sau3A I
Pst I

pL31

5'AATTCATGGGC|Sau3A
     GTACCCGCTAG|
          3'

T4 Ligase,
EcoR I, Pst I

Sau3A I
      *
5'AATTCATGGGC|GATC ... TGCA|3'
     GTACCCGCTAG| ... Pst I

Fig. 3

Fig. 4

0128467
4/5

Fig. 5

| | DOCUMENTS CONSIDERED TO BE RELEVANT | | EP 84106214.4 |
|---|---|---|---|

| Category | Citation of document with indication, where appropriate, of relevant passages | Relevant to claim | CLASSIFICATION OF THE APPLICATION (Int. Cl. ³) |
|---|---|---|---|
| D,X | NATURE, vol. 294, no. 5841, December 10, 1981 (London, New York) <br><br> H.M. SHEPARD et al.: "A single amino acid change in IFN-$\beta_1$ abolishes its antiviral activity" pages 563-565 <br><br> * Page 563, summary; fig. 2 * <br><br> -- | 1,19, 22,23 | C 07 C 103/52 <br> C 12 N 15/00 <br> C 12 P 21/00 <br> A 61 K 45/00 // <br> C 12 R 1/19 |
| A | EP - A2 - 0 042 246 (CANCER INSTITUTE OF JAPANESE FOUNDATION FOR CANCER RESEARCH) <br><br> * Claims 1,2,6,7 * <br><br> -- | 1,5 | |
| A,D | THE JOURNAL OF BIOLOGICAL CHEMISTRY vol. 257, no. 19, October 10, 1982, (Baltimore, USA) <br><br> E. REHBERG et al.: "Specific Molecular Activities of Recombinant and Hybrid Leucocyte Interferons" pages 11497-11502 <br><br> * Page 11497, summary; fig. 2 * <br><br> -- | 1,5 | **TECHNICAL FIELDS SEARCHED (Int. Cl. ³)** <br><br> C 07 C 103/00 <br> C 12 P <br> A 61 K <br> C 12 N |
| A | EP - A2 - 0 077 670 (GENENTECH., INC.) <br><br> * Abstract * <br><br> ---- | 1 | |

The present search report has been drawn up for all claims

| Place of search | Date of completion of the search | Examiner |
|---|---|---|
| VIENNA | 30-08-1984 | WOLF |